# EUROPÄISCHE PATENTSCHRIFT

(11) **EP 1 191 888 B1**
(45) Veröffentlichungstag und Bekanntmachung des Hinweises auf die Patenterteilung: **15.10.2003**
(21) Anmeldenummer: 00945840.7
(22) Anmeldetag: 28.06.2000
(51) Int. Cl.: A61B 17/28

(54) **MEDIZINISCHES, INSBESONDERE CHIRURGISCHES INSTRUMENT**
MEDICAL, ESPECIALLY SURGICAL, INSTRUMENT
INSTRUMENT MEDICAL, NOTAMMENT CHIRURGICAL

(30) Priorität: 01.07.1999 DE 19930426
(43) Veröffentlichungstag der Anmeldung: 03.04.2002
(73) Patentinhaber: Karl Storz GmbH & Co. KG, 78532 Tuttlingen (DE)
(72) Erfinder: BACHER, Uwe, D-78532 Tuttlingen (DE)
(74) Vertreter: Hofmeister, Frank Horst
(86) Internationale Anmeldenummer: EP0006006
(87) Internationale Veröffentlichungsnummer: WO01001870

(56) Entgegenhaltungen:
- EP-A- 0 596 272
- EP-A- 0 700 662
- DE-A- 2 033 247
- DE-A- 4 341 736
- DE-A- 19 514 098
- US-A- 4 309 042
- US-A- 5 893 851

## Beschreibung

Die Erfindung betrifft ein medizinisches, insbesondere chirurgisches Instrument, mit zwei über eine Kupplung verbindbaren Instrumententeilen, mit einer mit einem der beiden Instrumententeile verbundenen Kupplungshülse und einem von dem anderen Instrumententeil gebildeten, in der Kupplungshülse festlegbaren Kupplungsschaft, wobei die Kupplungshülse und der Kupplungsschaft über mindestens ein in einem Führungskäfig angeordnetes Kupplungselement kraftschlüssig miteinander verbindbar sind und wobei das Kupplungselement im wesentlichen nur axial beweglich ist und die Kupplung nur ein Federelement aufweist.

Ein gattungsgemäßes medizinisches Instrument ist beispielsweise aus der DE-A1 43 41 736 bekannt. Bei diesem bekannten Instrument erfolgt das Festlegen des Kupplungsschaftes an der Griffbranche bzw. der Hülse über eine Kugel als Kupplungselement, die im verrasteten Zustand Aufnahme in einer Vertiefung im Kupplungsschaft findet. Aufgrund dieser Konstruktion muß die gesamte auf das Instrument und über das Instrument ausgeübte axiale Kraft über die als Kupplungselement dienende Kugel übertragen werden. Dies führt zu extremen Belastungen der Vertiefung des Kupplungsschaftes und einer Querbelastung der Kugel an zwei nahe beieinander liegenden Punkten auf der Kugeloberfläche. DE-A1-4 341 736 korrespondiert zu dem Oberbegriff des ersten Anspruchs.

Ein weiteres medizinisches Instrument ist beispielsweise aus der DE-A1 195 14 098 der Anmelderin bekannt. Bei diesem als Rohrschaftinstrument ausgebildeten bekannten Instrument erfolgt das Festlegen der beiden Instrumententeile miteinander über einen in dem in die Kupplungshülse einzuführenden Kupplungsschaft ausgebildeten Einstich, in den im verrasteten Zustand das als Kugel ausgelegte Kupplungselement durch eine erste Feder druckbelastet eintritt. Zum Lösen der Verbindung ist die Kupplungshülse als um die Längsachse des Rohrschaftinstruments verdrehbare Drehhülse ausgebildet, wobei innerhalb der Drehhülse eine der Verdrehung entgegenwirkende zweite Federn nämlich eine Torsionsfeder angeordnet ist. Zwar ermöglicht die Kupplung dieses bekannten medizinischen Instruments eine sichere Verbindung der beiden Instrumententeile miteinander, jedoch ist der Durchmesser dieses Instruments unter anderem durch die Ausbildung der Drehhülse sehr groß, so daß ein solches Instrument beispielsweise zur Verwendung im HNO-Bereich zu groß und zu unhandlich ist. Darüber hinaus ist die Kupplung dieses Instruments in der Fertigung unter anderem aufgrund der Verwendung der beiden Federn sehr aufwendig und teuer.

Weitere mit Schnellkupplungen versehene medizinische Instrumente sind beispielsweise aus der DE-A1 39 34 610, der US-PS 4 403 959 sowie der US-PS 4 577 875 bekannt. All diese bekannten Instrumentenkupplungen weisen den Nachteil auf, daß sie einerseits einen großen Durchmesser aufweisen und andererseits wenigstens zwei Federelemente benötigen, um eine überwiegend spielfreie Verbindung der beiden Instrumententeile miteinander zu gewährleisten. Aufgrund dieses hohen konstruktiven Aufwands sind die bekannten mit Schnellkupplungen versehenen medizinischen Instrumente sehr teuer.

Ausgehend von diesem Stand der Technik liegt der Erfindung die **Aufgabe** zugrunde, ein medizinisches Instrument der eingangs genannten Art zu schaffen, welches einerseits eine im wesentlichen spielfreie Verbindung der beiden Instrumententeile miteinander gewährleistet und welches andererseits einfach und kostengünstig aufgebaut ist und sich durch eine schmale Bauweise auszeichnet.

Die **Lösung** dieser Aufgabenstellung ist erfindungsgemäß dadurch gekennzeichnet, daß das Kupplungselement an Anlaufschrägen von Kupplungshülse und Kupplungsschaft anliegt und der zwischen der Mittelachse der Kupplungshülse und der Anlaufschräge der Kupplungshülse gebildete spitze Winkel (α) größer ist als der zwischen der Mittelachse des Kupplungsschaftes und der Anlaufschräge des Kupplungsschaftes gebildete spitze Winkel (β).

Bei dem erfindungsgemäßen Instrument wird die Kraft beim Hereinschieben des Schaftes in die Kupplungshülse von den Auflagerflächen aufgenommen. Beim Herausziehen des Schaftes verriegelt sich dahingegen die Kupplung aufgrund der unterschiedlichen Steigungen der Anlaufschräge selbsttätig. Dabei wird die Kugel über zwei sich an der Kugeloberfläche nahezu gegenüberliegende Punkte als ganzes zusammengedrückt und stützt sich an den Anlaufschrägen von Kupplungshülse und Kupplungsschaft ab, was einen nur geringen Verschleiß zur Folge hat. Dieser Kupplungsaufbau mit den beiden Anlaufschrägen und dem zwischen diesen Anlaufschrägen angeordneten Kupplungselement ermöglicht einen einfachen Ausgleich von Fertigungstoleranzen, da im Gegensatz zum Stand der Technik gemäß der DE 43 41 736 A1 keine exakte Rastposition definiert wird. Die erfindungsgemäße Kupplung ermöglicht somit die Übertragung hoher Kräfte und Momente bei einer gleichzeitig sicheren Verbindung der Kupplungsteile.

Weiterhin wird mit der Erfindung vorgeschlagen, daß auf der Außenseite der Kupplungshülse ein in Axialrichtung der Kupplungshülse verstellbarer, mit dem Führungskäfig für das mindestens eine Kupplungselement im Eingriff stehender Schieber angeordnet ist, über den das mindestens eine Kupplungselement außer Eingriff mit der Anlaufschräge des Kupplungsschaftes verschiebbar ist. Durch die Verwendung des in Axialrichtung der Kupplungshülse verschiebbaren Schiebers ist es möglich den Durchmesser der Kupplung gegenüber den Kupplungen der aus dem Stand der Technik bekannten Instrumente deutlich zu verringern. Die Ausbildung des Schiebers ist darüber hinaus konstruktiv relativ einfach, was sich wiederum positiv auf die Herstellungskosten eines erfindungsgemäß ausgebildeten medizinischen Instruments auswirkt.

Gemäß einer praktischen Ausführungsform der Erfindung wird vorgeschlagen, daß der Schieber und der Führungskäfig für das mindestens eine Kupplungselement über mindestens ein Mitnahmeelement form- und/ oder kraftschlüssig miteinander verbunden sind. Über dieses Mitnahmeelement wird die Axialbewegung des Schiebers derart auf den Führungskäfig des Kupplungselements übertragen, daß durch das Verschieben des Schiebers entgegen der Kraft einer das Kupplungselement in die Raststellung druckbelastenden Druckfeder das mindestens eine Kupplungselement außer Eingriff der Anlaufschräge des Kupplungsschaftes bringbar ist.

Damit verhindert wird, daß sich die beiden Instrumententeile gegeneinander verdrehen können, wird erfindungsgemäß vorgeschlagen, daß zwischen den beiden Instrumententeilen eine Verdrehsicherung vorgesehen ist.

Gemäß einer ersten praktischen Ausführungsform der Erfindung ist die Verdrehsicherung als in einem der Instrumententeile angeordneter Zapfen ausgebildet, der in eine entsprechende Nut im anderen Instrumententeil eingreift.

Gemäß einer zweiten Ausführungsform der Erfindung ist der in einem der Instrumententeile ausgebildete Zapfen in jeweils einer von mehreren in dem anderen Instrumententeil ausgebildeten Nuten festlegbar. Durch diese Ausbildung ist es möglich, die Instrumententeile in verschiedenen Winkelstellungen zueinander festzulegen.

Die Möglichkeit, die Instrumententeile in verschiedenen Winkelstellungen zueinander festzulegen, kann gemäß einer weiteren Ausführungsform der Erfindung auch dadurch erzielt werden, daß die Verdrehsicherung als zwischen den Instrumententeilen wirkender Rastmechanismus ausgebildet ist.

Eine besonders gute Spielfreiheit zwischen den beiden miteinander zu verbindenden Instrumententeilen kann dadurch erzielt werden, daß sich der Kupplungsschaft über seine Umfangsfläche an zwei Auflagerflächen an der Innenseite der Kupplungshülse abstützt, deren Entfernung voneinander in axialer Richtung des Kupplungsschaftes betrachtet ein Mehrfaches des Durchmessers des Kupplungsschaftes beträgt. Durch die weit voneinander entfernten Auflagerflächen entstehen auch nicht so hohe Kippmomente auf das jeweils andere Instrumententeil, wie dies bei den bekannten Instrumenten der Fall ist.

Gemäß einer praktischen Ausführungsform der Erfindung ist eine der beiden Auflagerflächen konisch ausgebildet. Weiterhin wird mit der Erfindung vorgeschlagen, daß eine der beiden Auflagerflächen zylindrisch ausgebildet ist.

Bei der Verwendung eines erfindungsgemäßen medizinischen Instruments als Rohrschaftinstrument weist das andere Instrumententeil eine im Kupplungsschaft gelagerte Schub-/ Zugstange zur Betätigung eines medizinischen Greifinstruments auf.

Bei dieser Ausgestaltung ist das erfindungsgemäße medizinische Instrument dadurch gekennzeichnet, daß ein am proximalen Ende der Schub-/ Zugstange angeordnetes Halteelement in einem in axialer Richtung der Handhabe verschiebbaren Betätigungselement festlegbar ist.

Das Halteelement am proximalen Ende der Schub-/ Zugstange ist gemäß einer bevorzugten Ausführungsform der Erfindung als Kugel ausgebildet, die in eine entsprechend komplementär ausgebildete Kugelpfanne im Betätigungselement eingreift.

Schließlich wird mit der Erfindung vorgeschlagen, daß zum Verstellen der Schub-/ Zugstange zwischen dem verschwenkbaren Griffteil der Handhabe und dem axial in der Handhabe verschiebbaren Betätigungselement eine Zapfen-Schlitz-Steuerung ausgebildet ist.

Weitere Merkmale und Vorteile der Erfindung ergeben sich aus der nachfolgenden Beschreibung der zugehörigen Zeichnung, in der ein Ausführungsbeispiel eines erfindungsgemäßen medizinischen Instruments beispielhaft dargestellt ist. In der Zeichnung zeigt:
- Fig. 1a: eine teilweise geschnittene Seitenansicht eines erfindungsgemäßen medizinischen Instruments mit geschlossenem Greifinstrument;
- Fig. 1b: eine vergrößerte Detailansicht der Darstellung gemäß Fig. 1a;
- Fig. 2a: eine teilweise geschnittene Seitenansicht des medizinischen Instruments gemäß Fig. 1a, jedoch mit geöffnetem Greifinstrument;
- Fig. 2b: eine vergrößerte Detailansicht der Darstellung gemäß Fig. 2a;
- Fig. 3: eine teilweise geschnittene Seitenansicht der mit der Kupplungshülse versehenen Handhabe ohne das zweite Instrumententeil und
- Fig. 4: eine vergrößerte Detailansicht der Verbindung zwischen Kupplungshülse und Kupplungsschaft, das Verrasten der Kugel der Schub-/ Zugstange in der Kugelpfanne darstellend.

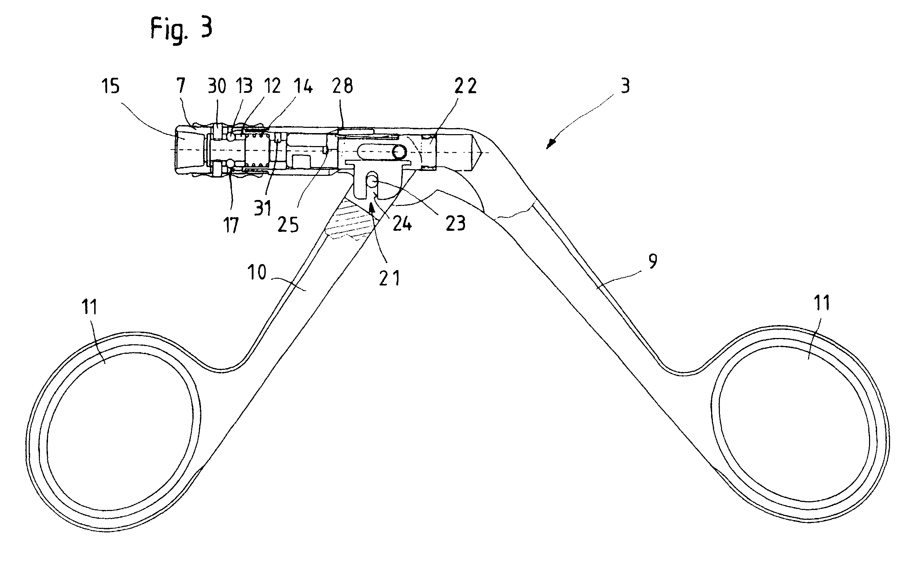

Bei dem in den Abbildungen dargestellten medizinischen Instrument handelt es sich um ein Rohrschaftinstrument, nämlich eine Faßzange. Dieses Instrument besteht aus zwei über eine Kupplung 1 miteinander verbundenen Instrumententeilen, nämlich dem Greifinstrument 2 und der Handhabe 3, die einzeln in Fig.3 dargestellt ist.

Das Greifinstrument besteht bei dem dargestellten Ausführungsbeispiel aus einem langgestreckten Schaft 4, an dessen distalem Ende das eigentliche Greifwerkzeug 5 angeordnet ist. Zum Verbinden mit der Handhabe 3 ist das proximale Ende des Schaftes 4 des Greifinstruments 2 als Kupplungsschaft 6 ausgebildet, der zusammen mit einer am distalen Ende der Handhabe 3 angeordneten Kupplungshülse 7 die Kupplung 1 bildet.

Das Betätigen des Greifwerkzeugs 5 erfolgt über eine in dem Schaft 4 gelagerte und über die Handhabe 3 betätigbare Schub-/ Zugstange 8.

Die Handhabe 3 besteht aus einem starren Griffteil 9 und einem gegenüber dem starren Griffteil 9 verschwenkbaren Griffteil 10. Zum Erfassen und Bedienen der Handhabe 3 sind an den freien Enden der Griffteile 9, 10 Fingerösen 11 ausgebildet.

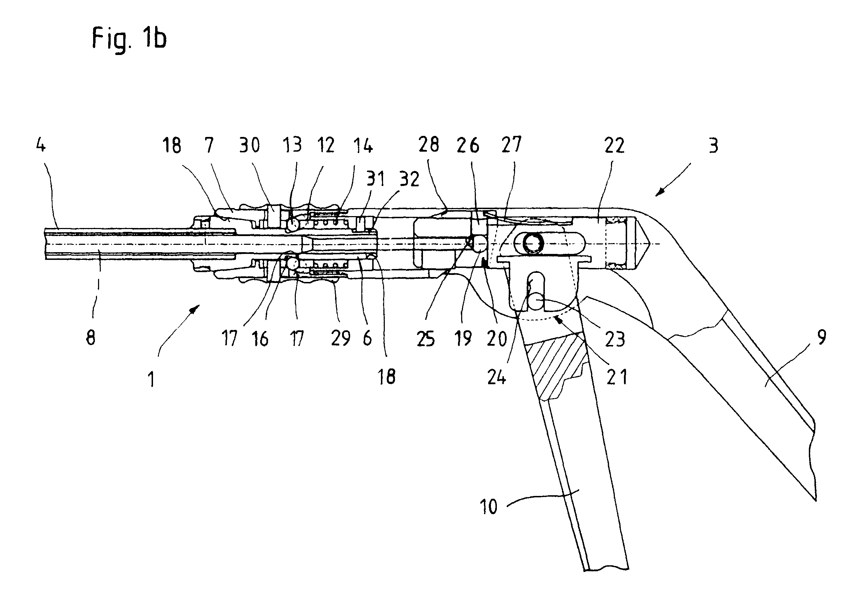

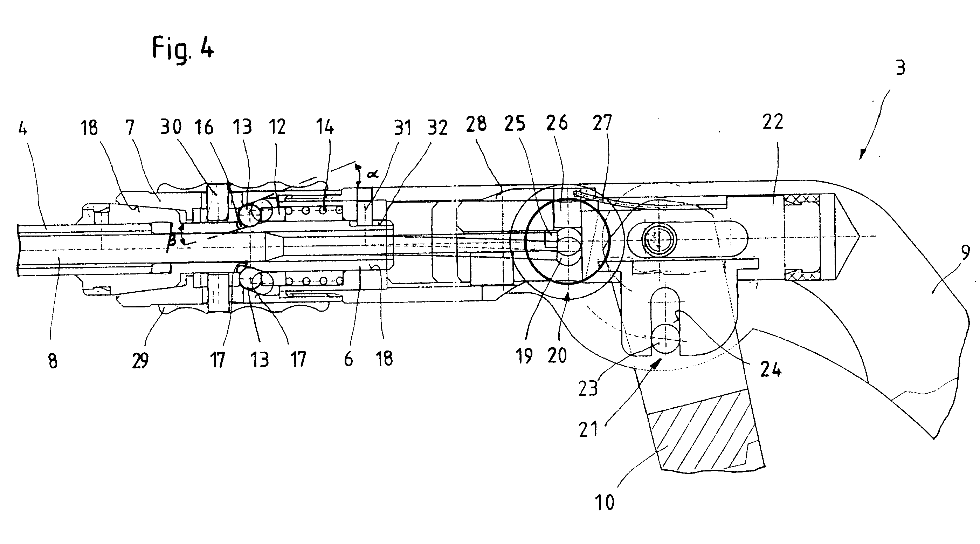

Der Aufbau der Kupplung 1 sowie die Arbeitsweise Handhabe 3 und Schub-/ Zugstange 8 ist den Abbildungen Fig. 1b, 2b, 3 und insbesondere Fig. 4 zu entnehmen.

Die bei dem dargestellten Ausführungsbeispiel mit der Handhabe 3 verbundene Kupplungshülse 7 weist auf der der Aufnahme des Kupplungsschaftes 6 dienenden Innenseite einen axialverschiebbaren Führungskäfig 12 auf, in dem als Kugeln ausgelegte Kupplungselemente 13 angeordnet sind. Der ringförmig ausgebildete Führungskäfig 12 ist über eine Druckfeder 14 in Richtung auf eine Aufnahmeöffnung 15 der Kupplungshülse 7 für den Kupplungsschaft 6 vorgespannt und weist einen freien Innendurchmesser zwischen den Kupplungselementen 13 auf, der kleiner ist als der Außendurchmesser des Kupplungsschaftes 6, so daß der Führungskäfig 12 mitsamt den darin gelagerten Kupplungselementen 13 zum proximalen Ende der Handhabe 3 hin verschoben wird, wenn der Kupplungsschaft 6 in die Kupplungshülse 7 eingesetzt-wird. Das Verrasten von Greifinstrument 2 und Handhabe 3 bzw. Kupplungsschaft 6 und Kupplungshülse 7 erfolgt dadurch, daß in der äußeren Mantelfläche des Kupplungsschaftes 6 ein umlaufender Einstich 16 ausgebildet ist, in den die Kupplungselemente 13 eintreten, sobald der Kupplungsschaft 6 ausreichend tief in die Kupplungshülse 7 eingeschoben worden ist.

In der Rastposition der Kupplungselemente 13 liegen die als Kugeln ausgebildeten Kupplungselemente 13 an Anlaufschrägen 17 an, die einerseits auf der Innenseite der Kupplungshülse und andererseits im Bereich des Einstichs 16 in den Kupplungsschaft 6 ausgebildet sind. Die Neigungen der Anlaufschrägen 17 zur Mittelachse von Kupplungshülse 7 und Kupplungsschaft 6 sind so ausgebildet, daß der zwischen der Mittelachse der Kupplungshülse 7 und der Anlaufschräge 17 der Kupplungshülse 7 gebildete Winkel α größer ist als der zwischen der Mittelachse des Kupplungsschaftes 6 und der Anlaufschräge 17 im Einstich 16 gebildete Winkel β. Aufgrund des Winkelunterschieds α > β und der Vorspannung der Kupplungselemente 13 über die an dem Führungskäfig 12 anliegende Druckfeder 14 stehen die beiden miteinander zu verbindenden Bauteile 2 und 3 an der konischen Auflagerfläche 18 immer unter Druck, was wiederum eine besonders gute und spielfreie Fixierung der beiden Bauteile 2 und 3 gegeneinander bewirkt.

Wie weiterhin insbesondere aus Fig. 4 ersichtlich ist, liegt der Kupplungsschaft 6 innerhalb der Kupplungshülse 7 an zwei Auflagerflächen 18 an, deren Abstand voneinander ein Mehrfaches des Außendurchmessers des Kupplungsschaftes 6 entspricht. Durch dieses weite Auseinanderliegen der Auflagerflächen voneinander ergibt sich ebenso ein geringeres Spiel zwischen Greifinstrument 2 und Handhabe 3, als dies bei den aus dem Stand der Technik bekannten Instrumenten der Fall ist. Unter Belastung bei geschlossenem Greifwerkzeug 5 ist die auf die Auflagerflächen 18 wirkende Kraft so groß, daß keine Torsion zwischen Handhabe 3 und Greifinstrument 2 möglich ist.

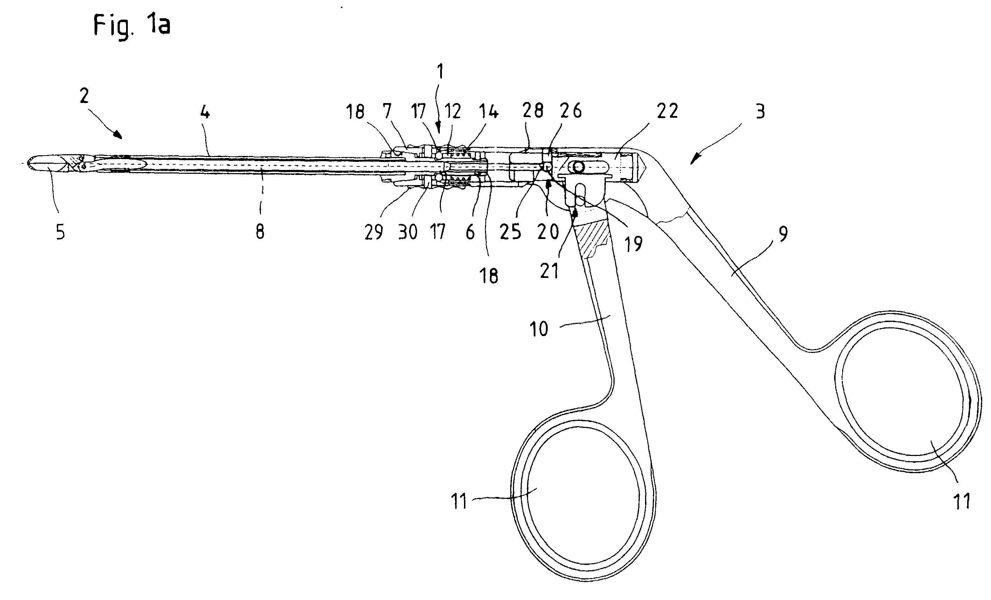

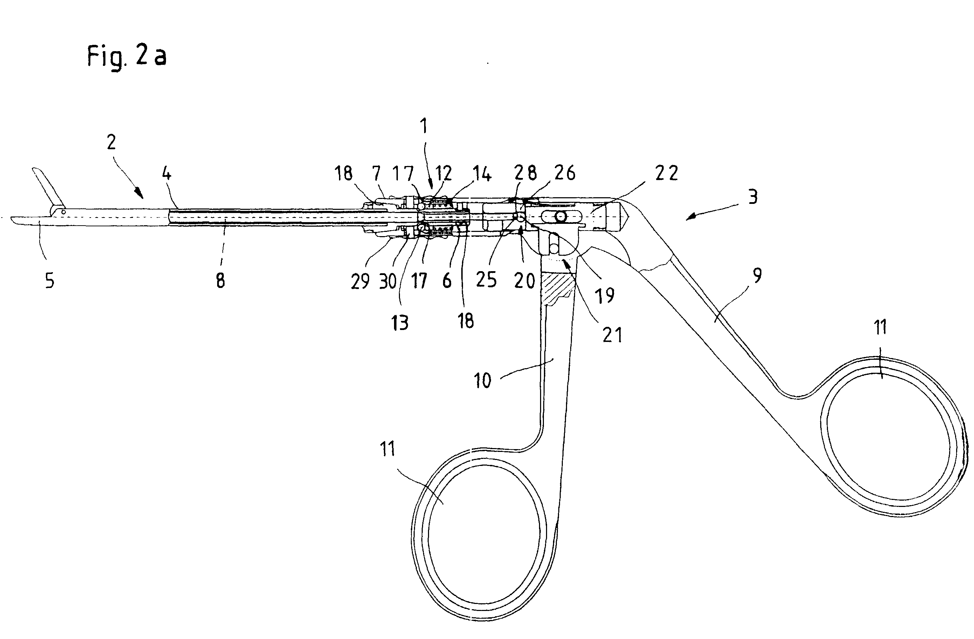

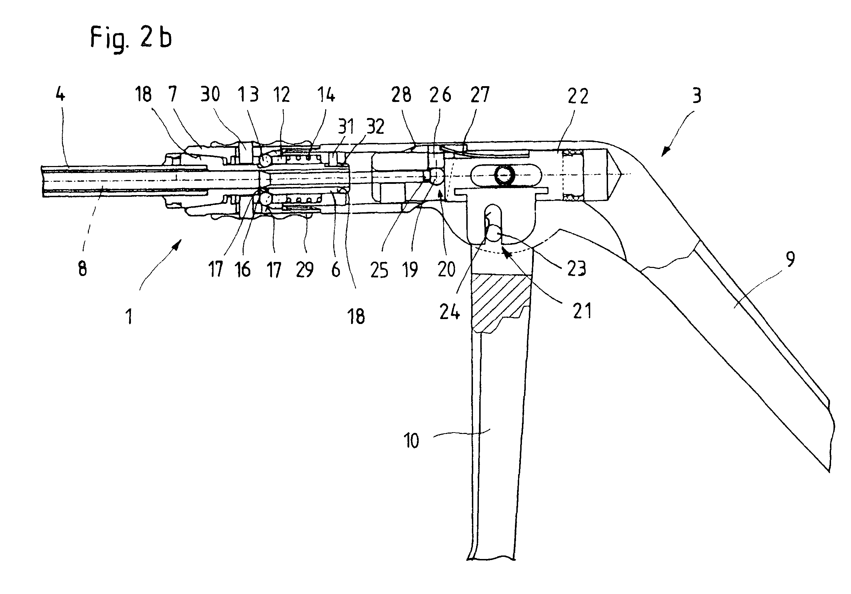

Zum Fixieren der Schub-/ Zugstange 8 an der Handhabe 3 ist am proximalen Ende der Schub-/ Zugstange 8 eine Kugel 19 angeordnet, die in einer Kugelpfanne 20 der Handhabe 3 festlegbar ist. Das Verstellen des Greifwerkzeugs 5 von der in Fig. 1a dargestellten geschlossenen Stellung in die in Fig. 2a dargestellte geöffnete Stellung erfolgt über eine Zapfen-Schlitz-Steuerung 21 zwischen dem verschwenkbaren Griffteil 10 der Handhabe 3 und einem Betätigungselement 22, das in axialer Richtung der Handhabe 3 verstellbar in der Handhabe 3 angeordnet ist. Im dargestellten Ausführungsbeispiel ist der Zapfen 23 der Zapfen-Schlitz-Steuerung 21 mit dem verschwenkbaren Griffteil 10 verbunden, während der Schlitz 24 in dem Betätigungselement 22 ausgebildet ist.

In der verriegelten Stellung in der Kugelpfanne 20 hintergreift die Kugel 19 ein Rastelement 25 und liegt an einem Entriegelungsbolzen 26 an, der über ein Federelement 27 fort von der Kugel 19 vorgespannt ist. Durch Verschieben des Betätigungselements 22 mittels des verschwenkbaren Griffteils 10 läßt sich das Betätigungselement 22 entlang einer auf der Innenseite der Handhabe 3 ausgebildeten Steuerkurve 28 verfahren.

Das Verriegeln und Entriegeln der Instrumententeile 2 und 3 über die Kupplung 1 sowie das Betätigen des Greifwerkzeugs 5 über die Handhabe 3 geschieht wie folgt:

Zum Ausbilden des in Fig. 1a und Fig. 2a dargestellten zusammengesetzten medizinischen Instruments wird in die Kupplungshülse 7 der in Fig.3 dargestellten Handhabe 3 das Greifinstrument 2 über den am proximalen Ende des Schaftes 4 des Greifinstruments 2 ausgebildeten Kupplungsschaft 6 eingesetzt. Da der Außendurchmesser des Kupplungsschaftes 6 größer ist als der freie Innendurchmesser zwischen den in dem ringförmigen Führungskäfig 12 angeordneten Kupplungselementen 13, wird der Führungskäfig 12 mitsamt der in dem Führungskäfig 12 gelagerten Kupplungselemente 13 entgegen der Kraft der Druckfeder 14 von der Aufnahmeöffnung 15 der Kupplungshülse 7 fort gedrückt, wenn der Kupplungsschaft 6 in die Kupplungshülse 7 eingeschoben wird.

Das Zurückdrücken des Führungskäfigs 12 geschieht so lange, bis der in der Mantelfläche des Kupplungsschafts 6 ausgebildete Einstich 16 in den Bereich der Kupplungselemente 13 gelangt. In diesem Moment treten die Kupplungselemente 13 in den umlaufenden Einstich 16 ein, wodurch für die Druckfeder 14 wieder die Möglichkeit besteht, den Führungskäfig 12 in Richtung der Aufnahmeöffnung 15 der Kupplungshülse zu drücken. Dies geschieht wiederum so lange, bis die Kupplungselemente 13 an den Anlaufschrägen 17 der Kupplungshülse 7 und des Einstichs 16 anliegen. Durch den Winkelunterschied zwischen den Anlaufschrägen 17 ergibt sich aufgrund des permanenten Drucks der Druckfeder 14 eine sichere und spielfreie Fixierung der beiden miteinander zu verbindenden Instrumententeile 2 und 3.

Zum Entriegeln der Verbindung ist auf der Außenseite der Kupplungshülse 7 ein in Axialrichtung der Kupplungshülse 7 verschiebbarer Schieber 29 angeordnet, über den mittels eines Mitnahmeelements 30 der Führungskäfig 12 entgegen der Kaft der Druckfeder 14 verschiebbar ist.

Beim Verschieben des Schiebers 29 hin zum proximalen Ende der Handhabe 3 wird über das Mitnahmeelement 30 der Führungskäfig 12 samt Kupplungselementen 13 mitgenommen. Aufgrund des Winkelunterschieds zwischen den Anlageflächen der Anlaufschrägen 17 wird der Abstand zwischen den beiden Anlageflächen wieder größer, bis der Kupplungsschaft 6 wieder aus der Kupplungshülse 7 entnommen werden kann. Das Kupplungselement 13 tritt dabei außer Eingriff mit dem Einstich 16, so daß die Kupplungsverbindung aufgehoben wird.

Beim Einsetzen des Kupplungsschafts 6 in die Kupplungshülse 7 wird darüber hinaus die in dem Schaft 4 des Greifinstruments 2 gelagerte Schub-/ Zugstange 8 nach unten gebogen, wie dies in Fig. 4 dargestellt ist. Dieses Verbiegen der Schub/ Zugstange 8 erfolgt, bis beim Einschieben des Kupplungsschafts 6 in die Kupplungshülse 7 die Kugel 19 der Schub-/ Zugstange 8 das Rastelement 25 überwunden hat und die Kugel 19 in der Kugelpfanne 20 fixierend hintergreift.

Das Betätigen des Greifwerkzeugs 5 erfolgt bei Betätigung des verschwenkbaren Griffteils 10 über die Zapfen-Schlitz-Steuerung 21, wodurch das an der Kugel 19 anliegende Betätigungselement 22 in axialer Richtung innerhalb der Handhabe 3 verschiebbar ist.

Um zu verhindern, daß sich die Handhabe 3 und das Greifinstrument 2 gegeneinander verdrehen können, ist zwischen diesen beiden Instrumententeilen 2, 3 eine Verdrehsicherung angeordnet, die beim dargestellten Ausführungsbeispiel aus einem in der Handhabe 3 gelagerten Zapfen 31 besteht, der in eine entsprechend ausgebildete Nut 32 im Kupplungsschaft 6 eingreift.

Neben der dargestellten Möglichkeit, die Instrumententeile 2, 3 starr verdrehsicher miteinander zu verbinden, besteht auch die nicht dargestellte Möglichkeit, die Instrumententeile 2, 3 beispielsweise über einen Rastmechanismus in verschiedenen Winkelstellungen zueinander festzulegen.

Wie aus den Abbildungen ersichtlich ist, erlaubt die beschriebene Konstruktion des medizinischen Instruments eine schlanke Bauweise mit einem geringen Aussendurchmesser, so daß ein solches Instrument auch bei Operationen im HNO-Bereich einsetzbar ist.

### Bezugszeichenliste

- 1: Kupplung
- 2: Greifinstrument
- 3: Handhabe
- 4: Schaft
- 5: Greifwerkzeug
- 6: Kupplungsschaft
- 7: Kupplungshülse
- 8: Schub-/ Zugstange
- 9: starrer Griffteil
- 10: verschwenkbarer Griffteil
- 11: Fingeröse
- 12: Führungskäfig
- 13: Kupplungselement
- 14: Druckfeder
- 15: Aufnahmeöffnung
- 16: Einstich
- 17: Anlaufschräge
- 18: Auflagerfläche
- 19: Kugel
- 20: Kugelpfanne
- 21: Zapfen-Schlitz-Steuerung
- 22: Betätigungselement
- 23: Zapfen
- 24: Schlitz
- 25: Rastelement
- 26: Entriegelungsbolzen
- 27: Federelement
- 28: Steuerkurve
- 29: Schieber
- 30: Mitnahmeelement
- 31: Zapfen
- 32: Nut

- α: Winkel
- β: Winkel

## Patentansprüche

1. Medizinisches Instrument, insbesondere chirurgisches Instrument, mit zwei über eine Kupplung verbindbaren Instrumententeilen (2, 3), wobei ein Instrumententeil als Handhabe (3) mit einem starren Griffteil (9) und einem gegenüber dem starren Griffteil (9) verschwenkbaren Griffteil (10) ausgebildet ist und wobei das andere Instrumententeil (2) eine in dem Kupplungsschaft (6) gelagerte Schub-/ Zugstange (8) zur Betätigung eines medizinischen Greifinstruments (2) aufweist, mit einer mit einem der beiden Instrumententeile (3) verbundenen Kupplungshülse (7) und einem von dem anderen Instrumententeil (2) gebildeten, in der Kupplungshülse (7) festlegbaren Kupplungsschaft (6), wobei die Kupplungshülse (7) und der Kupplungsschaft (6) über mindestens ein in einem Führungskäfig (12) angeordnetes Kupplungselement (13) kraftschlüssig miteinander verbindbar sind und wobei das Kupplungselement (13) im wesentlichen nur axial beweglich ist und die Kupplung nur ein Federelement (14) aufweist,
**dadurch gekennzeichnet,**
**daß** das Kupplungselement (13) an Anlaufschrägen (17) von Kupplungshülse (7) und Kupplungsschaft (6) anliegt und der zwischen der Mittelachse der Kupplungshülse (7) und der Anlaufschräge (17) der Kupplungshülse (7) gebildete spitze Winkel (α) größer ist als der zwischen der Mittelachse des Kupplungsschaftes (6) und der Anlaufschräge (17) des Kupplungsschaftes (6) gebildete spitze Winkel (β).

2. Medizinisches Instrument nach Anspruch 1, **dadurch gekennzeichnet, daß** auf der Außenseite der Kupplungshülse (7) ein in Axialrichtung der Kupplungshülse (7) verstellbarer, mit dem Führungskäfig (12) für das mindestens eine Kupplungselement (13) zumindest kraftschlüssig verbundener Schieber (29) angeordnet ist, über den das mindestens eine Kupplungselement (13) außer Eingriff mit der Anlaufschräge (17) des Kupplungsschaftes (6) verschiebbar ist.

3. Medizinisches Instrument nach Anspruch 2, **dadurch gekennzeichnet, daß** der Schieber (29) und der Führungskäfig (12) über mindestens ein Mitnahmeelement (30) form- und/ oder kraftschlüssig miteinander verbunden sind.

4. Medizinisches Instrument nach mindestens einem der Ansprüche 1 bis 3, **dadurch gekennzeichnet, daß**, um ein Verdrehen der beiden Instrumententeile (2, 3) gegeneinander zu verhindern, eine Verdrehsicherung vorgesehen ist.

5. Medizinisches Instrument nach Anspruch 4, **dadurch gekennzeichnet, daß** die Verdrehsicherung als in einem der Instrumententeile (3) angeordneter Zapfen (31) ausgebildet ist, der in eine entsprechende Nut (32) im anderen Instrumententeil (2) eingreift.

6. Medizinisches Instrument nach Anspruch 4, **dadurch gekennzeichnet, daß** die Verdrehsicherung als in einem der Instrumententeile (3) angeordneter Zapfen (31) ausgebildet ist, der in jeweils einer von mehreren in dem anderen Instrumententeil (2) ausgebildeten Nuten (32) festlegbar ist.

7. Medizinisches Instrument nach Anspruch 4, **dadurch gekennzeichnet, daß** die Verdrehsicherung als zwischen den Instrumententeilen (2, 3) wirkender Rastmechanismus ausgebildet ist.

8. Medizinisches Instrument nach mindestens einem der Ansprüche 1 bis 7, **dadurch gekennzeichnet, daß** sich der Kupplungsschaft (6) über seine Umfangsfläche an zwei Auflagerflächen (18) an der Innenseite der Kupplungshülse (7) abstützt, deren Entfernung voneinander in axialer Richtung des Kupplungsschaftes (6) betrachtet ein Mehrfaches des Durchmesseres des Kupplungsschaftes (6) beträgt.

9. Medizinisches Instrument nach Anspruch 8, **dadurch gekennzeichnet, daß** eine der beiden Auflagerflächen (18) konisch ausgebildet ist.

10. Medizinisches Instrument nach Anspruch 8 oder 9, **dadurch gekennzeichnet, daß** eine der beiden Auflagerflächen (18) zylindrisch ausgebildet ist.

11. Medizinisches Instrument nach mindestens einem der Ansprüche 1 bis 10, **dadurch gekennzeichnet, daß** ein am proximalen Ende der Schub-/ Zugstange (8) angeordnetes Halteelement in einem in axialer Richtung der Handhabe (3) verschiebbaren Betätigungselement (22) festlegbar ist.

12. Medizinisches Instrument nach Anspruch 11, **dadurch gekennzeichnet, daß** das Halteelement am proximalen Ende der Schub-/ Zugstange (8) als Kugel (19) ausgebildet ist, die in eine entsprechend komplementär ausgebildete Kugelpfanne (20) im Betätigungselement (22) eingreift.

13. Medizinisches Instrument nach Anspruch 11 oder 12, **dadurch gekennzeichnet, daß** zum Verstellen der Schub-/ Zugstange (8) zwischen dem verschwenkbaren Griffteil (10) der Handhabe (3) und dem axial in der Handhabe (3) verschiebbaren Betätigungselement (22) eine Zapfen-Schlitz-Steuerung (21) ausgebildet ist.

## Claims

1. Medical instrument, in particular a surgical instrument, with two instrument parts (2, 3) which can be connected via a coupling, one instrument part being designed as a handle (3) with a rigid grip part (9) and with a grip part (10) which can be pivoted relative to the rigid grip part (9), and the other instrument part (2) having a push/pull rod (8) mounted in the coupling shaft (6) for the purpose of actuating a medical gripping instrument (2), with a coupling sleeve (7) connected to one of the two instrument parts (3), and a coupling shaft (6) which is formed by the other instrument part (2) and can be fixed in the coupling sleeve (7), said coupling sleeve (7) and said coupling shaft (6) being able to be connected to one another with a force fit via at least one coupling element (13) arranged in a guide cage (12), and the coupling element (13) being movable substantially only in an axial direction, and the coupling having only one spring element (14), **characterized in that** the coupling element (13) bears on contact bevels (17) of coupling sleeve (7) and coupling shaft (6), and the acute angle (α) formed between the centre axis of the coupling sleeve (7) and the contact bevel (17) of the coupling sleeve (7) is greater than the acute angle (β) formed between the centre axis of the coupling shaft (6) and the contact bevel (17) of the coupling shaft (6).

2. Medical instrument according to Claim 1, **characterized in that** the outside of the coupling sleeve (7) is provided with a slide (29) which is adjustable in the axial direction of the coupling sleeve (7) and which is connected at least with a force fit to the guide cage (12) for the at least one coupling element (13), and via which slide (29) the at least one coupling element (13) can be disengaged from the contact bevel (17) of the coupling shaft (6).

3. Medical instrument according to Claim 2, **characterized in that** the slide (29) and the guide cage (12) are connected to one another with a form fit and/or force fit via at least one carrier element (30).

4. Medical instrument according to at least one of Claims 1 to 3, **characterized in that** a protection against twisting is provided in order to prevent twisting of the two instrument parts (2, 3) relative to one another.

5. Medical instrument according to Claim 4, **characterized in that** the protection against twisting is designed as a pin (31) which is arranged in one of the instrument parts (3) and which engages in a corresponding groove (32) in the other instrument part (2).

6. Medical instrument according to Claim 4, **characterized in that** the protection against twisting is designed as a pin (31) which is arranged in one of the instrument parts (3) and which can be fixed in one of several grooves (32) formed in the other instrument part (2).

7. Medical instrument according to Claim 4, **characterized in that** the protection against twisting is designed as a locking mechanism acting between the instrument parts (2, 3).

8. Medical instrument according to at least one of Claims 1 to 7, **characterized in that** the coupling shaft (6), about its peripheral surface, is supported on two bearing surfaces (18) on the inside of the coupling sleeve (7), the distance between the bearing surfaces (18), viewed in the axial direction of the coupling shaft (6), being a multiple of the diameter of the coupling shaft (6).

9. Medical instrument according to Claim 8, **characterized in that** one of the two bearing surfaces (18) is of conical design.

10. Medical instrument according to Claim 8 or 9, **characterized in that** one of the two bearing surfaces (18) is of cylindrical design.

11. Medical instrument according to at least one of Claims 1 to 10, **characterized in that** a retaining element arranged at the proximal end of the push/pull rod (8) can be fixed in an actuating element (22) which is displaceable in the axial direction of the handle (3).

12. Medical instrument according to Claim 11, **characterized in that** the retaining element at the proximal end of the push/pull rod (8) is designed as a ball (19) which engages in a complementary ball socket (20) in the actuating element (22).

13. Medical instrument according to Claim 11 or 12, **characterized in that** a pin-and-slot control (21) for adjusting the push/pull rod (8) is formed between the pivotable grip part (10) of the handle (3) and the actuating element (22) axially displaceable in the handle (3).

## Revendications

1. Instrument médical, en particulier instrument chirurgical, comportant deux parties d'instrument (2, 3) susceptibles d'être reliées via un accouplement, dans lequel une partie d'instrument est réalisée sous forme de manette (3) avec une partie de poignée rigide (9) et avec une partie de poignée (10) mobile en pivotement par rapport à la partie de poignée rigide (9), et l'autre partie d'instrument (2) comprend une barre poussée/tirée (8) montée dans le tronc d'accouplement (6) et destinée à l'actionnement d'un instrument de préhension médical (2), comportant une douille d'accouplement (7) reliée à l'une les deux parties d'instrument (3) et un tronc d'accouplement (6) formé par l'autre partie d'instrument (2) et susceptible d'être immobilisé dans la douille d'accouplement (7), la douille d'accouplement (7) et le tronc d'accouplement (6) étant susceptibles d'être reliés l'un à l'autre en coopération de forces via au moins un élément d'accouplement (13) agencé dans une cage de guidage (12), et dans lequel l'élément d'accouplement (13) est mobile sensiblement seulement axialement et l'accouplement ne comprend qu'un élément élastique (14),
**caractérisé en ce que** l'élément d'accouplement (13) s'appuie contre des pentes de montée (17) de la douille d'accouplement (7) et du tronc d'accouplement (6), et **en ce que** l'angle aigu (α) formé entre l'axe médian de la douille d'accouplement (7) et la pente de montée (17) de la douille d'accouplement (7) est supérieur à l'angle (β) formé entre l'axe médian du tronc d'accouplement (6) et la pente de montée (17) du tronc d'accouplement (6).

2. Instrument médical selon la revendication 1, **caractérisé en ce que** sur le côté extérieur de la douille d'accouplement (7) est agencé un tiroir (29) déplaçable en direction axiale de la douille d'accouplement (7) et relié au moins par coopération de forces avec la cage de guidage (12) pour ledit au moins un élément d'accouplement (13), tiroir via lequel ledit au moins un élément d'accouplement (13) est mobile en translation pour se dégager de la pente de montée (17) du tronc d'accouplement (6).

3. Instrument médical selon la revendication 2, **caractérisé en ce que** le tiroir (29) et la cage de guidage (12) sont reliés l'un à l'autre par coopération de formes et/ou de forces via au moins un élément entraîneur (30).

4. Instrument médical selon l'une des revendications 1 à 3, **caractérisé en ce qu'**il est prévu un blocage anti-rotation pour empêcher une rotation des deux parties d'instrument (2, 3) l'une par rapport à l'autre.

5. Instrument médical selon la revendication 4, **caractérisé en ce que** le blocage anti-rotation est réalisé sous forme de tenon (31) agencé dans l'une des parties d'instrument (3), qui s'engage dans une gorge correspondante (32) dans l'autre partie d'instrument (2).

6. Instrument médical selon la revendication 4, **caractérisé en ce que** le blocage anti-rotation est réalisé sous forme de tenon (31) agencé dans l'une des parties d'instrument (3), qui est susceptible d'être immobilisé dans une gorge respective parmi plusieurs gorges (32) ménagées dans l'autre partie d'instrument (2).

7. Instrument médical selon la revendication 4, **caractérisé en ce que** le blocage anti-rotation est réalisé sous forme de mécanisme d'enclenchement agissant entre les parties d'instrument (2, 3).

8. Instrument médical selon l'une des revendications 1 à 7, **caractérisé en ce que** le tronc d'accouplement (6) prend appui par sa surface périphérique sur deux surfaces de butée (18) sur le côté intérieur de la douille d'accouplement (7), dont la distance l'une de l'autre, vue en direction axiale du tronc d'accouplement (6), est un multiple du diamètre du tronc d'accouplement (6).

9. Instrument médical selon la revendication 8, **caractérisé en ce que** l'une des deux surfaces de butée (18) est réalisée conique.

10. Instrument médical selon l'une ou l'autre des revendications 8 et 9, **caractérisé en ce que** l'une des deux surfaces de butée (18) est réalisée cylindrique.

11. Instrument médical selon l'une des revendications 1 à 10, **caractérisé en ce qu'**un élément de retenue agencé à l'extrémité proximale de la barre poussée/tirée (8) est susceptible d'être immobilisé dans un élément d'actionnement (22) mobile en translation en direction axiale de la manette (3).

12. Instrument médical selon la revendication 11, **caractérisé en ce que** l'élément de retenue à l'extrémité proximale de la barre poussée/tirée (8) est réalisé sous forme de bille (19) qui s'engage dans une cavité à bille (20) réalisée de façon complémentaire dans l'élément d'actionnement (22).

13. Instrument médical selon l'une ou l'autre des revendications 11 et 12, **caractérisé en ce que** pour déplacer la barre poussée/tirée (8) entre la partie de poignée (10) mobile en pivotement de la manette (3) et l'élément d'actionnement (22) mobile en translation axialement dans la manette (3), il est prévu une commande à tenon-et-fente (21).
